Europäisches Patentamt

European Patent Office

Office européen des brevets

(19)

(11) **EP 0 577 511 B1**

(12) **FASCICULE DE BREVET EUROPEEN**

(45) Date de publication et mention
de la délivrance du brevet:
**30.08.2000 Bulletin 2000/35**

(51) Int. Cl.⁷: $G01N\ 29/02$, $G01N\ 33/04$, $G01N\ 29/20$

(21) Numéro de dépôt: **93401696.5**

(22) Date de dépôt: **30.06.1993**

(54) **Dispositif et procédé de détection de changement de phases d'un produit liquide, gelifié ou solide**

Verfahren und Vorrichtung zum Feststellen von Phasenveränderungen eines Produktes

Device and method for the detection of changes of phase of a product

(84) Etats contractants désignés:
**AT BE CH DE ES GB IT LI LU NL PT**

(30) Priorité: **03.07.1992 FR 9208221**

(43) Date de publication de la demande:
**05.01.1994 Bulletin 1994/01**

(73) Titulaire: **BONGRAIN S.A.**
**78283 Guyancourt Cédex (FR)**

(72) Inventeurs:
• **Beudon, Didier**
 **F-75116 Paris (FR)**
• **Audidier, Yves**
 **F-91370 Verrière le Buisson (FR)**
• **Frouin, André**
 **F-78000 Versailles (FR)**
• **Innocent, Jean-Pierre**
 **F-78000 Versailles (FR)**

(74) Mandataire: **Orès, Bernard et al**
 **Cabinet ORES**
 **6, Avenue de Messine**
 **75008 Paris (FR)**

(56) Documents cités:
 **WO-A-84/04167**      **DE-A- 1 473 707**
 **GB-A- 2 160 974**

## Description

L'invention se rapporte principalement à un dispositif et à un procédé de détection de changement de phases et de caractérisation de la phase d'un produit liquide, gélifié ou solide à son contact ou à travers son contenant.

FR-A-2 566 910 met en oeuvre un guide d'ondes ultrasonores traversant un milieu à étudier. L'atténuation des ondes ultrasonores entre un transducteur d'émission placé à une extrémité et un transducteur de réception placé à l'extrémité opposée, permet de déterminer les propriétés du milieu. La variation de l'atténuation des ondes reçues dans le guide d'ondes est une fonction de la variation de l'impédance acoustique du milieu et de sa différence par rapport à l'impédance acoustique du guide d'ondes, le rayonnement de ce dernier dans le milieu à étudier constituant les pertes provoquant cette atténuation.

De même, WO-A-8 404 167 décrit la détection de la variation de l'impédance acoustique d'un milieu. Cette mesure est effectuée par un barreau, par exemple en verre, dont une extrémité est munie d'un transducteur électroacoustique générant des ondes ultrasonores. Seule la propagation des ondes ultrasonores dans le barreau est étudiée, les ondes ultrasonores pénétrant dans le milieu n'étant pas détectées.

Il est connu d'effectuer des contrôles de qualité en cours de processus de fabrication, notamment par des techniciens spécialisés. Un changement de personnel peut entraîner un manque de fiabilité qui, dans certains cas, s'ajoute au manque de précision de ces méthodes qui ne sont pas utilisables sur un grand nombre de produits, et qui ne sont pas automatisables.

Les techniques de mesure des modifications de comportement physique sur un échantillon, du type mesure de viscosité, altèrent le produit dans certains cas et peuvent être difficilement implantables sur une ligne de production en continu. On connaît, d'autre part, des texturomètres à ultrasons, utilisant les propriétés de non-propagation des ondes ultrasonores transversales dans un milieu liquide pour déterminer un temps de prise. Le texturomètre de type connu ne peut pas donner un temps de décaillage du lait et ne fonctionne correctement que pour des produits de type béton.

Il existe, notamment dans l'industrie du lait, un besoin non satisfait de suivi non destructif de gélification dans des cuves industrielles pouvant contenir plusieurs milliers de litres de produit. Par exemple, les industries de lait classiques (caillé, pressuré ou acide), cherchent à déterminer le temps de prise et/ou le temps de décaillage avec précision pour optimiser et standardiser leurs productions.

L'industrie du lait qui exploite des procédés à base de rétenats recherche à détecter un temps de prise et un temps de chambrage avant d'effectuer le moulage.

Plus généralement, toute industrie qui utilise des transitions de phases cherche à quantifier, de façon précise, les étapes de leurs procédés au niveau de la transition des phases pour pouvoir mener des rétroactions dans un but de régulation et d'automatisation.

C'est par conséquent un but de la présente invention d'offrir un dispositif de détection non destructif d'un changement de phases d'un produit.

C'est également un but de la présente invention d'offrir un procédé non destructif de détection d'un changement de phases d'un produit.

C'est aussi un but de la présente invention d'offrir une chaîne de production d'un produit comportant des moyens de suivi d'évolution du produit ne risquant pas de compromettre les caractéristiques de ce produit, notamment, dans le cas d'un aliment, ses propriétés organoleptiques.

C'est également un but de la présente invention d'offrir un procédé de suivi de production non destructif.

C'est aussi un but de la présente invention d'offrir un dispositif de détection, à travers un contenant, de changement de phases d'un produit.

C'est également un but de la présente invention d'offrir un procédé de détection d'un changement de phases d'un produit à travers son contenant.

C'est aussi un but de la présente invention d'offrir un dispositif de caractérisation non destructif de la phase d'un produit.

C'est également un but de la présente invention d'offrir un procédé non destructif de caractérisation de la phase d'un produit.

C'est aussi un but de la présente invention d'offrir un dispositif de détection d'un changement de phases et/ou de caractérisation de la phase d'un produit qui ne soit pas perturbé par une variation de températures.

C'est également un but de la présente invention d'offrir un procédé de détection d'un changement de phases d'un produit et/ou de caractérisation de la phase d'un produit qui n'est pas perturbé par un changement de températures.

C'est aussi un but de la présente invention d'offrir un dispositif de détection d'un changement de phases et/ou de caractérisation de la phase d'un produit rapide fournissant des résultats de mesure en temps réel.

C'est également un but de la présente invention d'offrir un procédé de détection rapide d'un changement de phases et/ou de caractérisation rapide de la phase d'un produit susceptible de fournir un résultat de mesure en temps réel.

Ces buts sont atteints par la mesure de l'énergie ultrasonore rayonnée par un produit insonifié avec une impulsion ultrasonore, avantageusement longitudinale, en fonction du temps, et avantageusement, de la température. Par insonifier un corps, on entend engendrer dans ce corps des vibrations aux fréquences ultrasonores.

L'invention a principalement pour objet un procédé de détection d'un changement de phases et/ou de caractérisation de la phase d'un produit (16) comportant une étape d'insonification du produit avec des ondes ultrasonores et une étape de réception du rayonnement acoustique, caractérisé en ce qu'il comporte les étapes consistant à :

- disposer un transducteur d'émission/réception ou un transducteur d'émission et un transducteur de réception à l'extérieur d'un contenant du produit à étudier;
- assurer un couplage acoustique entre le ou les transducteur et le produit à étudier :
- analyser le signal reçu du produit à étudier et le comparer à un modèle ;
- en déduire la phase ou le mélange de phases du produit.

L'invention a également pour objet un procédé caractérisé en ce qu'il comporte une étape de sélection automatique d'une fenêtre temporelle dans le signal capté ayant le meilleur rapport signal/bruit.

L'invention a aussi pour objet un procédé caractérisé en ce que l'on insonifie le produit avec une onde ultrasonore en mode de vibration longitudinale.

L'invention a également pour objet un procédé caractérisé en ce qu'il comporte une étape de mesure de l'atténuation des ondes ultrasonores dans le produit en fonction du temps de réception, c'est-à-dire en fonction de la profondeur de pénétration des ondes ultrasonores dans le produit.

L'invention a aussi pour objet un procédé caractérisé en ce qu'il comporte une étape d'émission d'ondes ultrasonores en direction d'un miroir acoustique immergé dans le produit et une étape de réception des ondes ultrasonores réfléchies sur le miroir.

L'invention a également pour objet un procédé caractérisé en ce qu'il comporte des étapes consistant à :

- calculer le logarithme népérien de l'enveloppe du signal reçu :
- calculer la régression linéaire R du logarithme de l'enveloppe du signal reçu pour les diverses parties de cette enveloppe :
- déterminer la fenêtre temporelle de l'enveloppe correspondant au maximum du coefficient de régression linéaire R du logarithme de l'enveloppe ;
- déterminer l'absorption des ondes ultrasonores uniquement dans ladite fenêtre temporelle.

L'invention a aussi pour objet un procédé caractérisé en ce qu'il comporte une étape de comparaison de l'énergie reçue en fonction du temps à un modèle ou à une échelle d'étalonnage.

L'invention a également pour objet un procédé caractérisé en ce qu'il comporte une étape de comparaison du spectre de puissance des ondes captées à un modèle ou à une échelle d'étalonnage.

L'invention a aussi pour objet un procédé caractérisé en ce qu'il comporte des étapes consistant à :

a) numériser le signal reçu sur N points,
b) choisir une succession de n points du signal, n étant inférieur à N,
c) calculer de la transformée de Fourrier, de préférence par l'algorithme TFR, de la succession de n points,
d) mesurer l'amplitude du résultat de calcul de la transformée de Fourrier,
e) répéter les étapes b) à d) pour une pluralité de successions de points.

L'invention a aussi pour objet un procédé caractérisé en ce qu'il comporte une étape de mesure de la température et de correction des résultats en fonction de la valeur mesurée de la température.

L'invention a également pour objet un procédé caractérisé en ce qu'il comporte une étape de détection automatique de l'apparition d'un défaut dans un produit, notamment d'un défaut risquant de compromettre les propriétés organoleptiques d'un produit alimentaire.

L'invention a aussi pour objet un procédé de fabrication d'un produit, notamment d'un produit alimentaire, caractérisé en ce qu'il met en oeuvre un procédé de détection de changement de phases et/ou de caractérisation de phases d'un produit.

L'invention a également pour objet un procédé caractérisé en ce qu'il comporte une étape de détection automatique de l'apparition d'un défaut dans un produit, notamment d'un défaut risquant de compromettre les propriétés organoleptiques d'un produit alimentaire et une étape de rétroaction sur au moins un paramètre de fabrication pour faire disparaître ou, tout au moins diminuer, les conséquences du défaut détecté.

L'invention a aussi pour objet un procédé caractérisé en ce qu'il comporte les étapes consistant à :

- mélanger deux produits ;
- déterminer la proportion des produits, morceaux ou air (foisonnement), ou les propriété physiques du mélange obtenu, notamment la viscosité, par le procédé selon l'invention ;
- modifier les proportions des produits pour obtenir le mélange désiré.

L'invention a également pour objet un procédé caractérisé en ce qu'il comporte une étape de prise de gel d'un produit dont la durée ainsi que la température à laquelle est porté le produit sont asservies par un procédé selon l'invention.

L'invention a aussi pour objet un dispositif pour la mise en oeuvre d'un procédé caractérisé en ce qu'il comporte une sonde d'émission et de réception ultrasonore, disposée(s) à l'extérieur d'un contenant du produit à étudier et reliée(s) à un émetteur pour générer un signal électrique converti en une onde ultrasonore par la sonde et un récepteur des ondes ultrasonores captées par la sonde ainsi que des moyens de traitement du signal pour détecter un changement de phases et/ou pour caractériser la phase d'un produit.

L'invention a également pour objet un dispositif caractérisé en ce qu'il comporte une sonde unique d'émission et de réception susceptible de recevoir l'énergie rétro-diffusée par le produit insonifié.

L'invention a aussi pour objet un dispositif caractérisé en ce qu'il comporte une sonde unique d'émission de réception ainsi qu'un miroir acoustique pour réfléchir vers la sonde des ondes ultrasonores.

L'invention a également pour objet un dispositif caractérisé en ce qu'il comporte une sonde d'émission des ondes ultrasonores et une sonde de réception des ondes ultrasonores placées en vis à vis de part et d'autre du produit à insonifier.

L'invention a aussi pour objet un dispositif pour la mise en oeuvre d'un procédé comportant une sonde d'émission et de réception ultrasonore reliée(s) à un émetteur pour générer un signai électrique converti en une onde ultrasonore par la sonde et un récepteur des ondes ultrasonores captées par la sonde ainsi que des moyens de traitement du signal pour détecter un changement de phases et/ou pour caractériser la phase d'un produit, caractérisé en ce que lesdits moyens de traitement du signal comportent des moyens de sélection automatique d'une fenêtre temporelle ayant le meilleur rapport signal/bruit.

L'invention sera mieux comprise au moyen de la description ci-après et des figures annexées sur lesquelles :

- la figure 1 est un schéma d'un premier exemple de réalisation d'un dispositif selon la présente invention ;
- la figure 2 est un schéma d'une sonde susceptible d'être mise en oeuvre dans le dispositif selon la présente invention ;
- la figure 3 est un schéma d'un second exemple de réalisation d'un dispositif selon la présente invention ;
- la figure 4 est un schéma illustrant une première disposition d'une sonde susceptible d'être mise en oeuvre dans le procédé selon la présente invention ;
- la figure 5 est un schéma illustrant une deuxième disposition de la sonde susceptible d'être mise en oeuvre dans le procédé selon la présente invention ;
- la figure 6 est un schéma illustrant une troisième disposition de la sonde susceptible d'être mise en oeuvre dans le procédé selon la présente invention ;
- la figure 7 est un schéma illustrant une quatrième disposition de la sonde susceptible d'être mise en oeuvre dans le procédé selon la présente invention ;
- la figure 8 est un schéma illustrant une cinquième disposition de la sonde susceptible d'être mise en oeuvre dans le procédé selon la présente invention ;
- la figure 9 est un schéma illustrant une sixième disposition de la sonde susceptible d'être mise en oeuvre dans le procédé selon la présente invention ;
- la figure 10 est un schéma illustrant une septième disposition de la sonde susceptible d'être mise en oeuvre dans le procédé selon la présente invention ;
- la figure 11 est un schéma illustrant une huitième disposition de la sonde susceptible d'être mise en oeuvre dans le procédé selon la présente invention ;
- la figure 12 est une courbe expérimentale d'un signal capté par le dispositif selon la présente invention ;
- la figure 13 est une courbe d'une enveloppe obtenue par calcul à partir de la courbe de signal de la figure 12 ;
- la figure 14 est un schéma d'un premier exemple de réalisation d'un dispositif d'élaboration d'enveloppes susceptibles d'être mises en oeuvre dans le dispositif selon la présente invention ;
- la figure 15 est un schéma d'un deuxième exemple de réalisation d'un dispositif d'élaboration d'enveloppes susceptibles d'être mises en oeuvre dans le dispositif selon la présente invention ;
- la figure 16 est un schéma illustrant la sommation d'une pluralité d'enveloppes ;
- les figures 17 à 20 illustrent les traitements mathématiques du signal capté ;
- la figure 21 est une courbe illustrant le signal obtenu en présence d'un défaut dans la cuve de mesure pour un traitement dans l'espace temporel ;
- la figure 22 est une courbe de référence dans l'espace temporel pour une cuve ne présentant pas de défauts ;
- la figure 23 illustre une courbe correspondant au cas de la figure 21 traitée dans l'espace des fréquences ;
- la figure 24 illustre une courbe dans l'espace des fréquences correspondant au cas de la figure 22 ;
- la figure 25 est un schéma d'un premier exemple de réalisation d'un dispositif d'asservissement industriel mettant en oeuvre un procédé selon la présente invention ;
- la figure 26 est une courbe illustrant l'évolution du procédé de fabrication de la figure 25 en fonction du temps ;

- les figures 27 à 30 illustrent les résultats de mesure du dispositif selon la présente invention en divers points du processus de la figure 26 ;
- la figure 31 est un organigramme du procédé des figures 25 et 26 ;
- la figure 32 est un schéma d'un deuxième exemple de réalisation d'un dispositif susceptible de mettre en oeuvre le procédé selon la présente invention ;
- la figure 33 est une courbe illustrant les mesures effectuées durant le déroulement du procédé mis en oeuvre par le dispositif de la figure 32 ou le montage de la figure 8 sur produit diffuseur ;
- la figure 34 est un organigramme du procédé des figures 32 et 33 ;
- les figures 35 et 36 illustrent les résultats de mesure en divers points de la courbe de la figure 26 après la prise de gel.

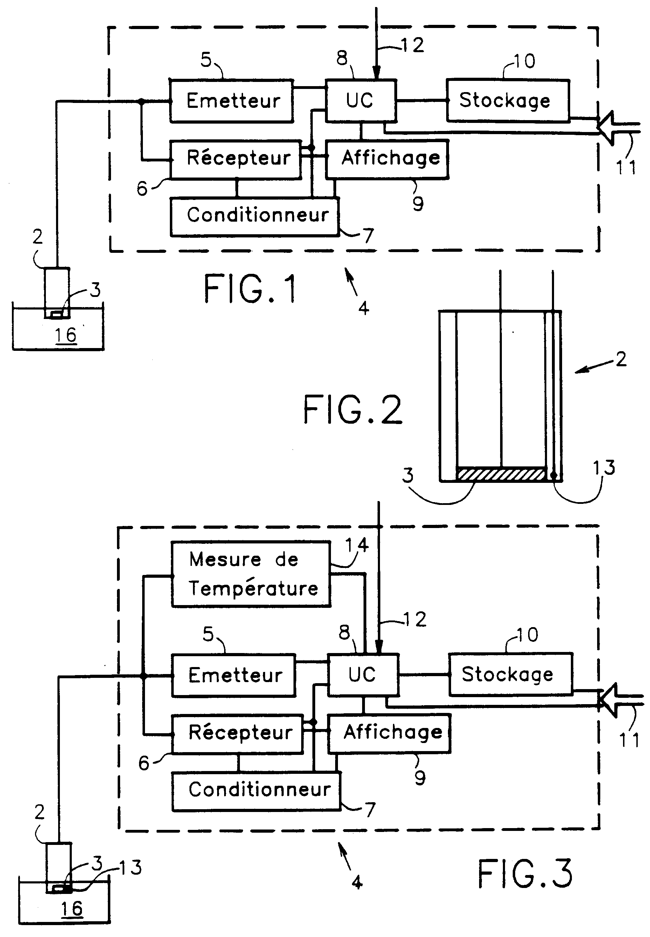

Sur les figures 1 à 36, on a utilisé les mêmes références pour désigner les mêmes éléments.

Dans la plupart des cas, les divers milieux rayonnent des bruits acoustiques caractéristiques lorsqu'ils sont insonifiés avec des ultrasons, notamment avec des impulsions brèves d'ondes ultrasonores en mode de vibration longitudinal. A certains changements de phases, le milieu peut devenir diffuseur d'ultrasons, sa texture peut alors être caractérisée par l'absorption des ultrasons en fonction du temps. De même, l'absorption des ultrasons pour une épaisseur de produit peut dépendre de sa phase. Lors de la transition de la phase, une amplification du bruit rayonné et l'analyse de l'absorption permettent de connaître les proportions du produit appartenant à chacune des phases. Un étalonnage du dispositif, selon la présente invention, permet d'identifier les phases ou les mélanges de phases des divers produits.

On traite le signal recueilli, la puissance moyenne et/ou les enveloppes du signal, et/ou l'on compare l'absorption des ultrasons à une référence, par exemple à l'absorption des ultrasons dans une même épaisseur d'eau pour caractériser la ou les phase(s) du produit insonifié.

Lors de l'installation d'un dispositif de détection de changement de phases ou de caractérisation de la phase d'un produit selon l'invention, par exemple sur une chaîne de production, l'homme de l'art effectue des essais pour déterminer quel est le procédé de traitement de signal des ondes ultrasonores captées par une sonde le plus caractéristique de la transition de phases à surveiller. Par exemple, l'homme du métier envoie, avant et après le changement de phases, des impulsions ultrasonores dans le milieu à surveiller, et compare l'influence du changement de phases sur la répartition spectrale du bruit capté, la décroissance des enveloppes du signal avec la profondeur de pénétration ou l'énergie ultrasonore totale captée.

L'homme de métier choisit, avantageusement, le traitement du signal dont la réponse est la plus affectée par le changement de phases.

Avantageusement, l'on associe un procédé de détection automatique du changement de phases adapté au milieu à surveiller et au procédé de traitement du signal choisi.

Dans un premier exemple, l'on mesure le nombre de passages successifs de l'amplitude du signal capté, par un seuil positif et un seuil négatif par unité de temps. Avantageusement, la valeur absolue du seuil positif est égale à la valeur absolue du seuil négatif. La présence de ces seuils est destinée à éliminer les oscillations du signal dues au bruit de mesure. Selon les valeurs du nombre de passages successifs par ces seuils, l'on se trouve avant, pendant ou après la transition de phases.

Dans un deuxième exemple de réalisation, l'on intègre l'énergie du signal dépassant un seuil par unité de temps. La valeur mesurée, après étalonnage, permet de déterminer la phase du produit surveillé.

Dans un troisième exemple, l'on compare l'énergie captée à la fréquence de la sonde, par exemple égale à 5 MHz, avec l'énergie captée à d'autres fréquences correspondant au bruit de mesure.

Le rapport signal/bruit permet de déterminer la phase du produit surveillé.

Dans un quatrième exemple, l'on effectue une analyse temps/fréquence du signal capté.

L'on effectue la transformée de Fourrier rapide glissante et l'on détermine les amplitudes du signal à diverses fréquences. Par exemple, sur un signal numérisé sur 4096 points, l'on effectue des transformées de Fourrier rapides sur des intervalles caractéristiques de 128 points. Ces intervalles de 128 points peuvent se recouvrir partiellement.

Dans chaque intervalle, l'on détermine l'atténuation en dB par unité de profondeur de pénétration et par unité de bande passante, par exemple en dB/(cm x MHz).

Dans un cinquième exemple, l'on compare l'amplitude de l'atténuation d'une enveloppe moyenne du signal à des valeurs d'étalonnage permettant de déterminer si l'on est avant, pendant (et à quel moment) ou après le changement de phases.

Il est bien entendu que l'utilisation simultanée de plusieurs procédés de mesure ou de caractérisation automatique de la phase d'un produit, ne sort pas du cadre de la présente invention.

De même, un ou plusieurs procédés mettant en oeuvre l'insonification avec les ondes ultrasonores longitudinales, peuvent être complétés par l'insonification avec des ondes ultrasonores transversales et/ou par une mesure de la vitesse de propagation des ondes acoustiques dans le milieu surveillé ou avec d'autres types de mesures.

L'étalonnage dépend, pour un procédé de fabrication donné, par exemple d'un fromage, non seulement du traitement mathématique du signal capté, mais également de la manière dont ce signal a été capté. Par exemple, les données d'étalonnage seront différentes pour une mesure d'atténuation en fonction de la profondeur de pénétration d'un signal rétrodiffusé et pour la mesure d'atténuation d'échos réfléchis sur un miroir (la profondeur de la pénétration étant égale à la profondeur du miroir).

Le choix de la configuration de la ou des sonde(s) et de l'éventuel miroir dépendent des caractéristiques acoustiques du milieu, avant, pendant et après le changement de phases.

Sur la figure 1, on peut voir un exemple de réalisation d'un dispositif, selon la présente invention, comportant une sonde 2 comprenant un transducteur électroacoustique 3 et une unité 4 de génération de traitement du signal. L'unité 4 comporte un émetteur d'ultrasons 5, un récepteur d'ultrasons 6, un circuit de conditionnement du signal 7, une unité centrale 8, un dispositif d'affichage 9 et avantageusement un dispositif de stockage des données 10 ainsi qu'une liaison de communication 11. Les éléments 2 à 11 sont avantageusement des dispositifs disponibles dans le commerce. La sonde 2 est avantageusement une sonde à transducteur 3 piézoélectrique servant à l'émission et à la réception de type plane mono-élément, du type décrit dans le Brevet français 87 07795, ou comportant une pluralité de transducteurs piézoélectriques alignés ou formant un secteur permettant la focalisation du faisceau. La résolution spatiale de la sonde et notamment sa fréquence nominale dépendent de la taille de la structure du produit étudié. Le transducteur 3 engendre des vibrations ultrasonores longitudinales dans le milieu à étudier.

L'émetteur 5 et le récepteur 6 peuvent être regroupés dans un émetteur-récepteur d'ultrasons du commerce, l'émetteur générant par exemple des impulsions de $\pm$ 100 V ou plus sous la forme d'impulsions de durées quasi nulles (Dirac), d'impulsions ou de train d'ondes. La fréquence d'échantillonnage du récepteur est supérieure ou égale à 5 fois la fréquence nominale de la sonde.

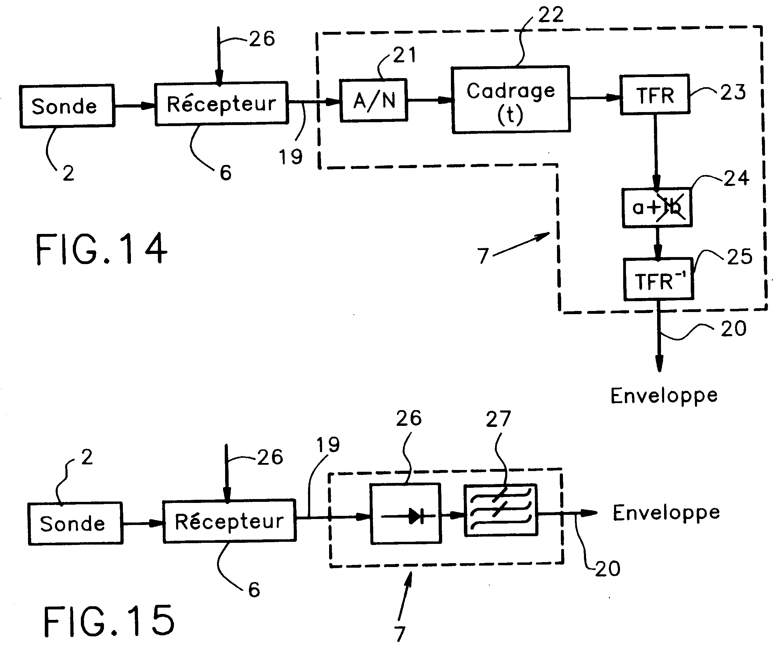

Deux exemples des circuits de conditionnement du signal 7 sont illustrés sur les figures 14 et 15.

L'unité centrale 8 est un micro-ordinateur réalisé, par exemple, sous forme d'une carte VME travaillant, par exemple, sous système d'exploitation UNIX (marque déposée), un ordinateur personnel fonctionnant sur le système d'exploitation MS-DOS (marque déposée) ou un automate industriel.

Lors d'essais d'installation, la Demanderesse a découvert que, contrairement à l'opinion généralement admise, les signaux électriques radiofréquences destinés à engendrer les ultrasons ou générés par la capture des ultrasons par la sonde 2 peuvent être transmis par un câble sur des distances pouvant atteindre et même dépasser une centaine de mètres. On a, par exemple, utilisé avec succès un câble coaxial blindé (3 conducteurs coaxiaux) d'une centaine de mètres pour relier la sonde 2 à l'unité 4. Dans un tel cas, l'on peut disposer l'unité 4 dans un endroit protégé, par exemple dans un local, ce qui permet de mettre en oeuvre un ordinateur de bureau qui ne supporterait pas l'environnement hostile d'une chaîne de production. Avantageusement, l'on met en oeuvre une sonde étanche susceptible de fonctionner dans un environnement hostile et d'être nettoyée facilement et complètement pour préserver l'hygiène de la chaîne de production.

De même, il est possible de séparer l'émetteur et le récepteur de l'unité centrale en les reliant par un câble. La liaison peut être réalisée aussi bien en analogique qu'en numérique et peut éventuellement être multiplexée.

Dans un premier exemple de réalisation, le dispositif d'affichage 9 comporte uniquement des afficheurs numériques ou alphanumériques indiquant les valeurs mesurées, des étapes de cycle de production et/ou des alarmes. Les alarmes visuelles peuvent éventuellement être complétées par des alarmes sonores. On utilise, par exemple, des afficheurs à cristaux liquides à diodes électro-luminescentes ou un tube à rayons cathodiques associé à un circuit de commande.

Dans une variante de réalisation, le dispositif d'affichage 9 comporte un écran graphique associé à une carte d'affichage.

L'unité de stockage de données 10 comporte, par exemple, un disque dur, un lecteur de disquettes ou des mémoires électroniques permanentes de type mémoires dynamiques (RAM en terminologie anglo-saxonne) secourues, des mémoires mortes programmables effaçables électriquement (EEPROM en terminologie anglo-saxonne) ou des mémoires à bulles.

Avantageusement, l'on stocke les données de mesure sous un format standard de tableur ou de base de données pour un traitement ultérieur.

La liaison de communication 11 comporte, par exemple, un port série RS-232.

L'unité centrale 8 est reliée à tous les autres éléments de l'unité 4 de génération et de traitement de signal. Elle reçoit, d'autre part, un signal de commande externe 12.

L'émetteur 5 et le récepteur 6 sont reliés à la sonde 2 d'émission/réception. Dans une variante de réalisation, l'émetteur 5 est relié à une sonde d'émission 2' tandis que le récepteur 6 est relié à une sonde de réception 2''. Le récepteur est relié au circuit de conditionnement du signal 7 et au dispositif d'affichage 9. Le circuit de conditionnement du signal 7 est relié au dispositif d'affichage 9. Le dispositif de stockage 10 est avantageusement relié par un accès direct mémoire (DMA en terminologie anglo-saxonne) à la liaison de communication 11.

Pour la mise en oeuvre d'un procédé selon la présente invention, l'on effectue un étalonnage préalable, par exemple sur des produits donnant satisfaction et sur des produits ne donnant pas satisfaction, et l'on implémente dans l'unité 4 de génération et de traitement du signal les consignes envoyées aux divers automates programmables de la chaîne de production et/ou les messages ou les informations à afficher.

L'étalonnage mis en oeuvre peut être soit individuel pour chaque machine ou pour chaque chaîne de production, soit global pour certains types de produits.

Une variation de température peut fausser les résultats de mesure selon l'invention en modifiant la correspondance entre le bruit ou l'énergie ultrasonore reçue et une phase ou un mélange de phases d'un produit. Pour des mesures qui devraient être effectuées dans une plage de température restreinte, un étalonnage initial du dispositif selon l'invention s'avère suffisant.

Par contre, pour pouvoir effectuer des mesures dans une large gamme de température, il s'avère avantageux de stocker dans l'unité centrale 8 ou dans l'unité de stockage 10 une table de corrections à apporter pour chaque plage des températures. Les largeurs des plages de températures assignées aux diverses tables, et donc le nombre de tables, dépendent de la précision désirée.

Le choix de la table à appliquer est soit effectué manuellement par l'opérateur qui communique par l'intermédiaire d'un clavier ou d'un commutateur la température du corps dont on veut déterminer la phase, soit, avantageusement, automatiquement par des moyens d'acquisition de la température connectés à l'unité centrale 8 de l'unité 4 de gestion et de traitement du signal. Sur la figure 2, l'on peut voir un exemple de sonde 2 comportant un dispositif de mesure de températures, par exemple un thermocouple 13. Dans l'exemple illustré sur la figure 2, le point sensible, c'est-à-dire la soudure chaude du thermocouple, est situé à proximité du transducteur 3 sur la face avant de la soude 2. Avantageusement, la paroi de la sonde 2 est réalisée en acier inoxydable.

La sonde 2 de la figure 2 est reliée à une unité 4 de génération et de traitement du signal de la figure 3 par un premier ensemble de conducteurs électriques véhiculant les impulsions à émettre et/ou les signaux reçus et un second ensemble de conducteurs véhiculant un signal comportant une information sur la température du corps dont on veut connaître la phase vers un dispositif de mesure de température 14 (de type connu) de l'unité 4 de génération et de traitement du signal de la figure 3. Avantageusement, les conducteurs reliés à l'émetteur 5 et au récepteur 6 d'une part, et ceux reliés au dispositif de mesure de température 14 sont, d'autre part, regroupés dans un câble unique.

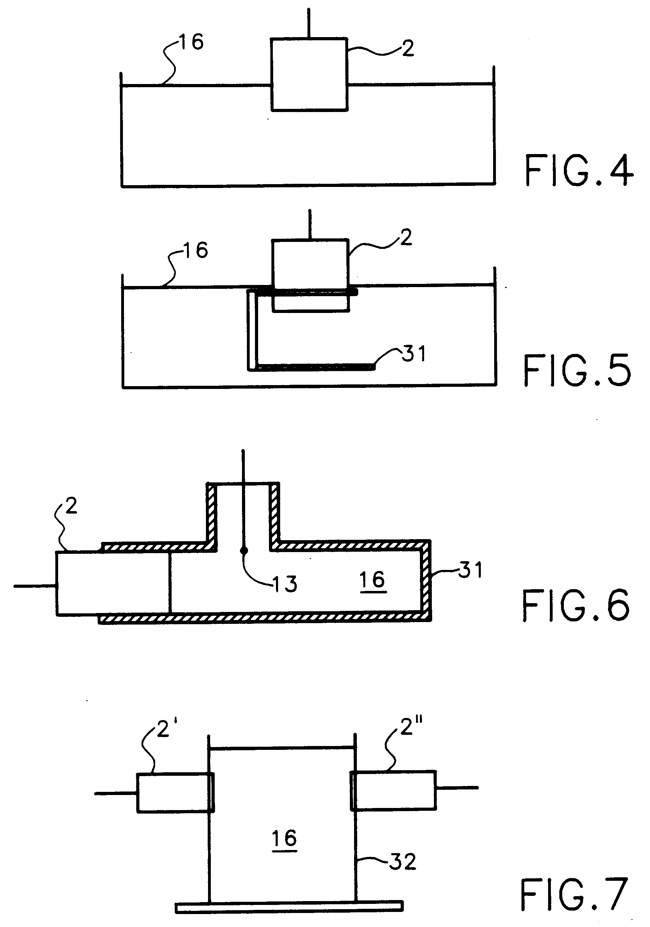

Diverses dispositions de la sonde 2 par rapport au produit 16 dont on veut suivre les modifications de la texture sont illustrés sur les figures 4 à 11.

Sur la figure 4, la sonde 2 est directement plongée dans la cuve d'un produit 16 diffuseur des ultrasons. La sonde unique 2 d'émission et de réception émet une impulsion ultrasonore dans le produit 16 et recueille l'énergie rétro-diffusée par ce produit.

Sur la figure 5, la sonde d'émission-réception 2 est plongée dans la cuve du produit 16, en étant équipée d'un miroir acoustique 31 réfléchissant les ondes ultrasonores en direction de la sonde 2.

Sur la figure 6, l'on peut voir une cellule de mesure équipée d'une sonde d'émission-réception 2 placée en face d'un miroir acoustique 31 et comportant des moyens de mesure de température 13 comme par exemple un thermocouple.

La cellule de mesure 32 de la figure 7 est équipée d'une sonde d'émission 2' et d'une sonde de réception 2" placées en vis à vis.

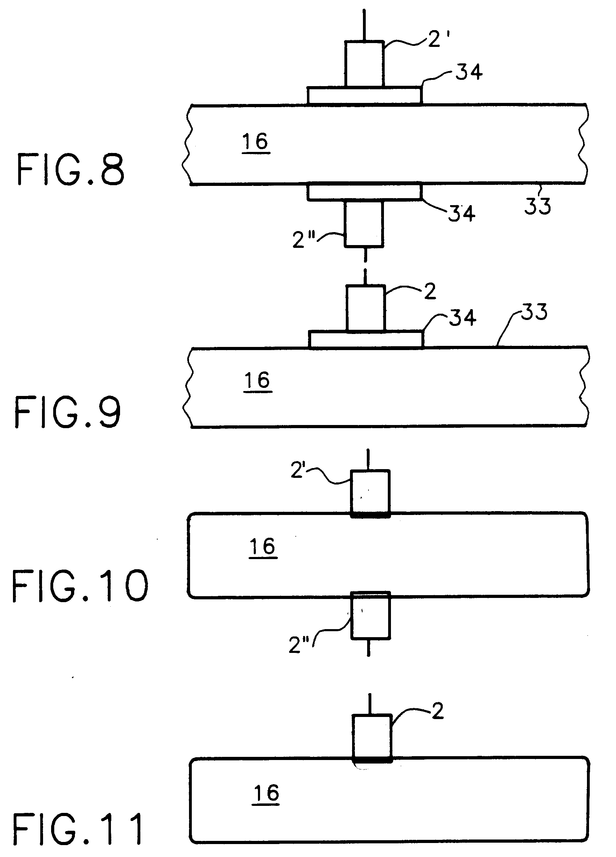

Sur la figure 8, l'on peut voir une sonde d'émission d'ultrasons 2' solidarisée en vis à vis d'une sonde de réception 2" sur une tuyauterie 33 par des sabots de fixation 34 jouant avantageusement leur rôle de coupleur acoustique.

Sur la figure 9, l'on peut voir une sonde 2 d'émission-réception solidarisée par un sabot 34 jouant avantageusement le rôle d'un coupleur acoustique avec tuyauterie 33 contenant un produit 16.

Sur la figure 10, l'on peut voir une sonde d'émission 2' et une sonde de réception 2" placées en vis à vis sur le produit 16.

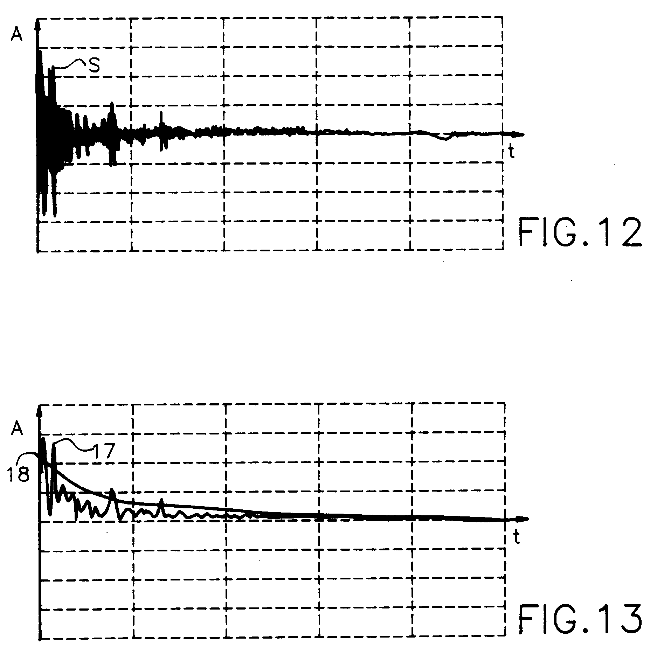

Sur la figure 11, une sonde 2 d'émission-réception est directement placée sur un produit, par exemple sur un fromage.

La disposition de la ou des sonde(s) de mesure dépend du produit étudié et des conditions opératoires. Si le produit 16 est diffuseur des ultrasons, l'on captera avantageusement sur un signal ultrasonore rétro-diffusé. Avec un produit non diffuseur d'ultrasons, l'on travaillera en transmission ou en réflexion sur un miroir. Le choix de la sonde tient compte du fait que le produit 16 est en agitation ou qu'il est statique.

Sur les figures 12, 13, 16 à 22, 27 à 30, 35 et 36 l'on a porté le temps en abscisse et l'amplitude du signal reçu en ordonné.

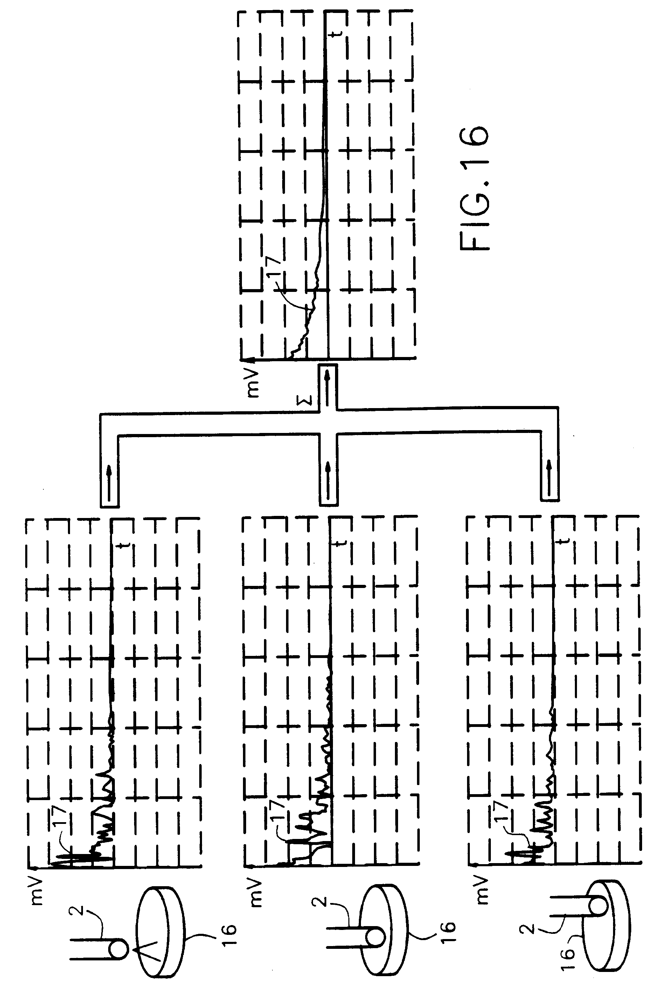

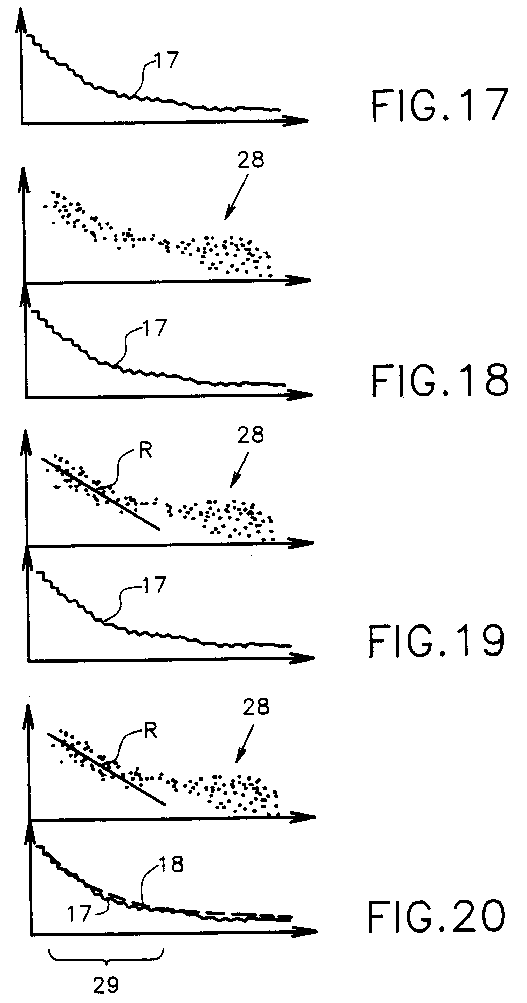

Sur la figure 16, l'on peut voir le calcul d'une enveloppe moyenne et, sur les figures 17 à 20, les diverses étapes de traitement susceptibles d'être mises en oeuvre sur les produits diffuseurs d'ultrasons pour un procédé selon la présente invention.

Sur la figure 16, l'on peut voir l'étape de calcul de la moyenne des enveloppes du signal reçu. La sonde 2 est placée dans une première position par rapport à un produit 16 dont on veut suivre l'évolution de la texture. Après émission d'une impulsion ultrasonore, l'on recueille un signal rétro-diffusé qui, traité par le circuit conditionneur 7, devient une première enveloppe 17. L'on répète la mesure pour une position différente de la sonde 2 par rapport au produit 16 et ainsi de suite jusqu'à l'obtention du nombre d'enveloppes 17 désiré, par exemple jusqu'à l'obtention de 100 enveloppes. L'on calcule la moyenne de toutes les valeurs 17, pour obtenir une enveloppe moyenne utilisée pour la suite du traitement.

Cette enveloppe moyenne 17 est illustrée sur la figure 17. On calcule le logarithme 28 de l'enveloppe moyenne 17, tels qu'illustrés sur la figure 18.

Sur la figure 19, l'on calcule la régression linéaire R du logarithme 28. La régression linéaire est de la forme y = mx + b , m étant la pente de la régression linéaire, c'est-à-dire l'atténuation des ondes ultrasonores et b étant l'ordonné à l'origine, c'est-à-dire le niveau de l'énergie diffusée, pour un nuage de N points, R étant le coefficient de la régression linéaire.

Dans l'exemple de réalisation avantageux, l'on calcule tout d'abord la régression linéaire sur N1 points, N1 étant par exemple égal à N/10. L'on calcule le coefficient de régression $R_1$ pour ce premier ensemble de points. L'on ajoute un incrément de points à l'ensemble de points sur lequel l'on effectue le calcul, cet incrément étant par exemple égal à N/10 et l'on calcule un second coefficient de régression linéaire $R_2$. L'on ajoute de nouveau un incrément des points à l'ensemble des points traités et l'on recalcule un nouveau coefficient de régression linéaire $R_3$ et ainsi de suite jusqu'à ce que l'on ait atteint les N points mesurés. Le nombre de points pour lequel le coefficient de régression linéaire est maximal donne le nombre de points utiles de la moyenne de l'enveloppe et le coefficient de régression linéaire de cet ensemble de points donne l'atténuation. Il est à noter que la présence de trous dans un fromage entraîne une perte de précision dans la mesure de l'atténuation. La sélection au maximum du coefficient de régression linéaire permet d'obtenir des mesures acceptables. Toutefois, la valeur maximale du coefficient de régression linéaire dans le cas d'un fromage comportant des trous sera plus faible, par exemple égale à 94 %. La valeur du coefficient de régression linéaire, au-dessus de la valeur minimale est le reflet des hétérogénéités de la pâte pour une longueur d'ondes donnée de la sonde à ultrasons.

Sur la figure 20, on a représenté une enveloppe modélisée 18 qui est une exponentielle décroissante dont le coefficient est proportionnel à l'absorption modélisée.

Lors d'une détection de phases, l'émetteur 5 génère une impulsion moyenne tension qui arrive au transducteur 3 de la sonde 2 ou 2' qui engendre une impulsion de vibrations mécaniques du transducteur 3 aux fréquences ultrasonores par exemple comprises entre 0,05 et 50 MHz. Les vibrations mécaniques se propagent éventuellement à travers un coupleur acoustique 34 dans un corps 16 dont on veut suivre l'évolution de la texture, par exemple dans un fromage en cours de fabrication ou à la fin de celle-ci. Lorsqu'on envoie une impulsion mécanique de durée $\tau$, la sonde capte un signal S haute fréquence dont l'amplitude varie avec le temps, telle qu'illustrée sur la figure 12 pour les produits diffuseurs des ultrasons. Ce signal est converti par le transducteur 3 de la sonde 2 ou 2' en un signal électrique de même allure qui est transmis au récepteur 6. La partie intéressante du signal correspondant à un décalage temporel par rapport à l'émission est transmise par le récepteur 6 au circuit de conditionnement du signal 7 qui en extrait une enveloppe 17, telle qu'illustrée sur la figure 13. D'autre part, on a représenté sur la figure 13 une courbe 18 de l'atténuation qui est un résultat intermédiaire de traitement et dont l'évolution est caractéristique d'un changement de phases.

Un premier exemple de circuit 7 de conditionnement du signal est illustré sur la figure 14. Ce circuit 7 comporte connecté en série entre une entrée 19 et une sortie 20, un convertisseur analogique/numérique 21, un circuit de cadrage temporel 22, un circuit de calcul de la transformée de Fourier rapide 23 (FFT en terminologie anglo-saxonne), un circuit 24 d'élimination de composants imaginaires du spectre de puissance et un circuit de calcul de la transformée de Fourrier rapide inverse (FFT$^{-1}$ en terminologie anglo-saxonne).

L'entrée 19 du circuit 7 de conditionnement du signal est connectée à la sortie du récepteur 6. Le convertisseur analogique/numérique 21 assure la numérisation du signal reçu. Le dispositif de cadrage temporel 22 envoie un signal de commande 26 au récepteur 6 déterminant le début et la fin de la transmission des signaux reçus vers les circuits 7. Les circuits de calcul de la transformée de Fourier rapide 23 effectuent les calculs de la transformée de Fourier discrète (par l'algorithme de calcul de la transformée de Fourier rapide) et délivrent un spectre de puissance du signal au circuit 24. Le circuit 24 effectue un filtrage numérique éliminant les composants imaginaires du spectre de puissance. Le circuit de calcul de la transformée de Fourier inverse 25 effectue le calcul de transformée de Fourier discrète inverse délivrant une enveloppe temporelle 17 de la figure 13.

Sur la figure 15, l'on peut voir un second exemple de réalisation d'un circuit 7 de conditionnement du signal comportant, connecté en série, un circuit 26 redresseur du signal et un filtre passe-bas 27.

Le circuit redresseur de signal, connecté à la sortie du récepteur 6, assure l'élimination des signaux correspondant à une amplitude négative. Le filtre passe-bas 27 assure l'élimination des hautes et moyennes fréquences.

D'une part, une mesure fiable et fidèle nécessite la mise en oeuvre d'un dispositif ayant un bon rapport signal/bruit.

D'autre part, l'utilisation en milieu industriel suppose un traitement automatique de l'information sans que l'opérateur, typiquement un technicien sans qualification particulière en traitement du signal ou en instrumentation, n'ait à calibrer l'appareil pour chaque mesure ou série de mesures.

Avantageusement, le dispositif selon la présente invention effectue un cadrage temporel de l'intervalle significatif de l'enveloppe 17 de la figure 13 en éliminant les parties de cette courbe qui représente un bruit acoustique et/ou électrique important, ce qui permet d'effectuer un calcul précis et fiable de l'atténuation des ultrasons.

Lors de la fabrication de certaines spécialités fromagères, comme par exemple pour la spécialité fromagère vendue sous la marque "ST MORET", l'apparition d'un défaut est caractérisée de façon plus efficace par une variation de l'amplitude en fonction de la fréquence de par une variation de l'amplitude du signal en fonction du temps de réception.

Si l'on ne remédie pas immédiatement au défaut, la spécialité fromagère fabriquée aura des caractéristiques organoleptiques inacceptables pour le consommateur ou tout au moins ne répondant pas aux critères de qualité du fabricant.

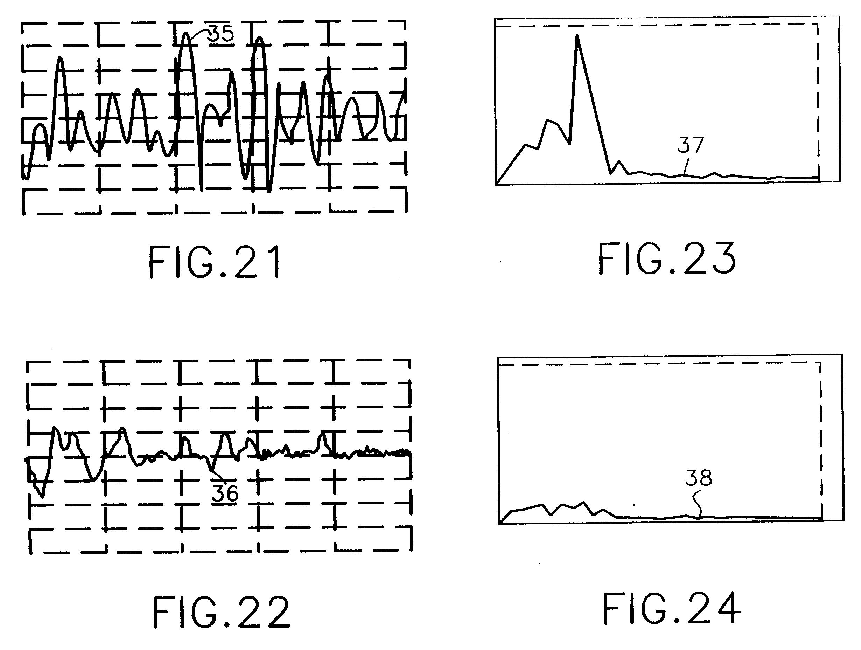

Sur la figure 21, l'on peut voir une courbe 35 représentant l'amplitude du signal reçu en fonction du temps pour un mélange servant la préparation d'une spécialité fromagère qui a été mal dosée et qui ne conduira pas à un produit acceptable.

Sur la figure 22, l'on peut voir la courbe correspondant à un mélange normal correspondant à un produit de très bonne qualité. La différence entre les courbes 35 et 36 est réelle et exploitable. Toutefois, la différence est beaucoup plus nette entre la courbe 37 de la figure 23 et la courbe 38 de la figure 24 représentant respectivement l'amplitude en fonction de la fréquence du mélange de la figure 21 et de la figure 22. L'analyse spectrale est, par exemple, effectuée sur le spectre de l'énergie reçue obtenue par une transformée de Fourier discrète du signal reçu ou, avantageusement, par un traitement temps/fréquence du signal, ce qui permet de réduire la puissance de calcul nécessaire.

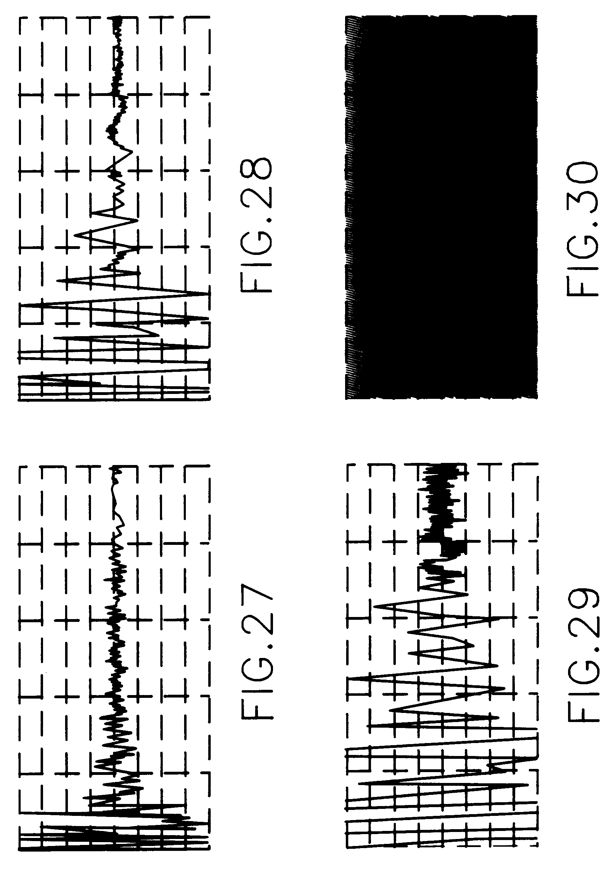

Sur la figure 25, l'on peut voir un appareil destiné à la prise de gel d'un mélange laitier, par exemple sous forme de pâte, en cours de fabrication, d'une spécialité fromagère ou d'un dessert lacté, comportant une cuve 39 équipée d'un dispositif de chauffage 40 et d'un mélangeur 70.

Une sonde 2 à ultrasons et un dispositif de mesure de température 13 sont reliés à une unité 4 de génération et de traitement du signal commandant un module 41 de régulation de température de la cuve 39. La prise de gel nécessite une montée de la température, un palier après la texturation et une descente en température. L'évolution de la température en fonction du temps, en cours de la prise de gel, est illustrée par la courbe 42 de la figure 26.

Sur les figures 27 à 30, l'on a utilisé la même échelle de temps et d'amplitude.

Sur la figure 27, l'on peut voir le signal rayonné par la pâte au point 43 de la courbe 42 au début du processus de gélification. La pâte ne diffuse pas les ultrasons.

Sur la figure 28, l'on peut voir le signal rayonné par la pâte au point 44. Au point 44, la pâte commence à rétrodiffuser des ondes ultrasonores.

Sur la figure 29, l'on peut voir le signal rayonné par la pâte au point 45 qui correspond au début de la texturation de la pâte. L'amplitude du signal rétro-

diffusé devient importante.

Sur la figure 30, l'on peut voir le signal rayonné par la pâte au point 46.

Dans la mesure où la pâte gélifiée est devenue une structure rigide et diffuseur d'ultrasons, le niveau du signal dépasse l'échelle d'amplitude choisie pour les figures 27 à 30, ce qui est symbolisé par des hachures sur la figure 30.

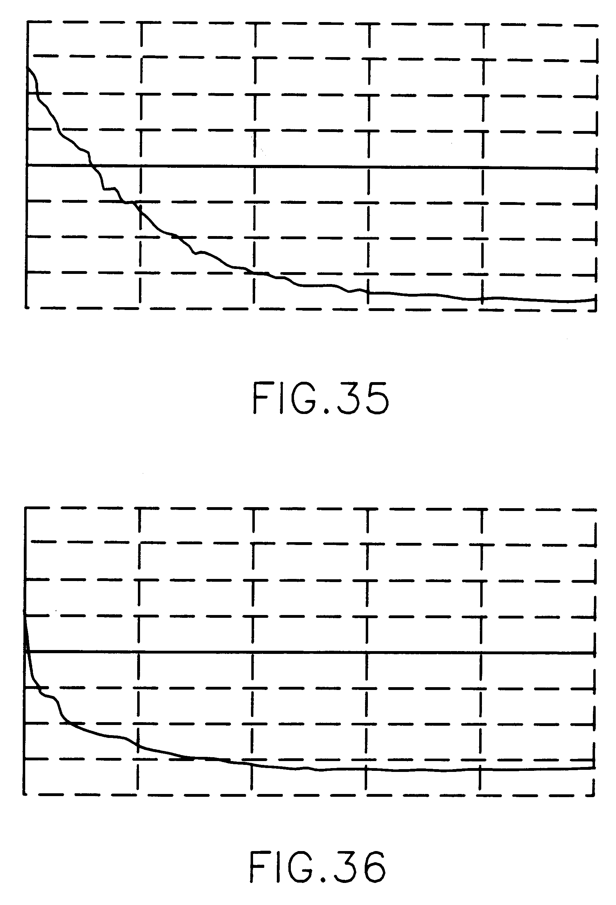

Sur la figure 35, l'on peut voir l'enveloppe du signal au point 46 de la figure 26 représentée avec une échelle plus grande en ordonné, permettant de visualiser des signaux de grandes amplitudes. Sur la figure 36, l'on peut voir (à la même échelle que sur la figure 35) l'enveloppe du signal au point 71 de la figure 26 correspondant sensiblement à la fin du cycle de production.

La méthode connue de gélification comporte trois étapes successives : une montée en température, un palier à une température donnée et un moulage.

L'on effectue la montée à température jusqu'à ce que l'on atteigne une température prédéfinie.

Les techniciens expérimentés estiment le moment le plus propice pour passer au moulage.

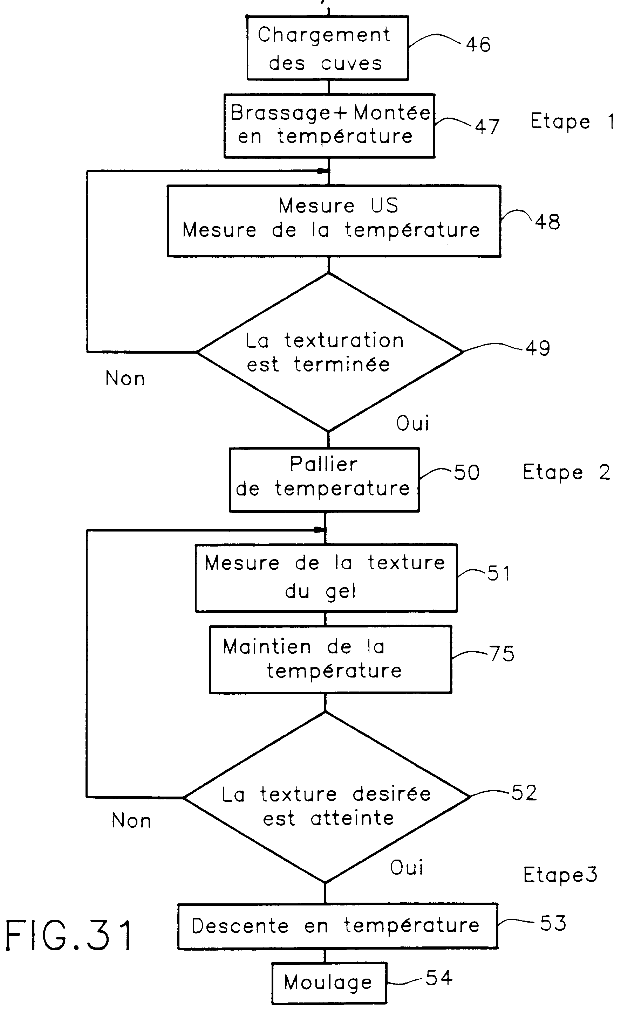

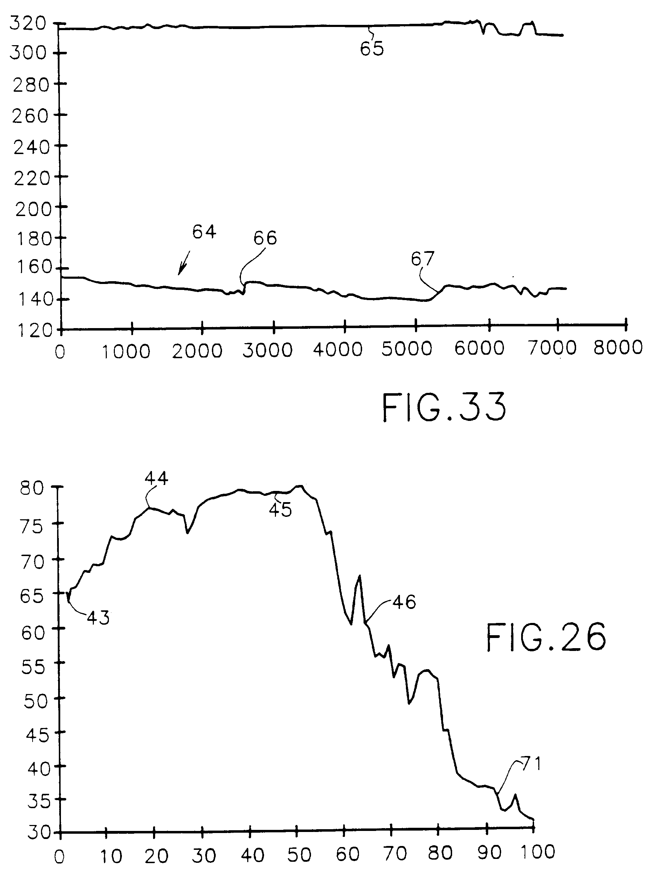

Un organigramme du procédé selon la présente invention est illustré sur l'organigramme de la figure 31.

En 46, l'on charge la cuve 39 avec la pâte 16.

On va en 47.

En 47, l'on effectue un brassage de la pâte 16 et une montée en température (chauffage).

On va en 48.

En 48, l'on mesure le niveau du bruit ultrasonore rétro-diffusé par la pâte 16, l'on mesure la température et l'on quantifie la texturation atteinte.

On va en 49.

En 49, l'on vérifie si l'on a atteint la texturation désirée.

Si non, on va en 48.

Si oui, on va en 50.

En 50, l'on effectue un palier à la dernière température mesurée.

On va en 51.

En 51, l'on mesure la texture du gel obtenu par mesure de l'atténuation des ultrasons.

On va en 75.

En 75, l'on assure le maintien en température pendant un incrément de temps.

On va en 52.

En 52, l'on vérifie si la texturation désirée est atteinte.

Si non, on va en 51.

Si oui, on va en 53.

En 53, l'on effectue une descente en température.

On va en 54.

En 54, l'on effectue le moulage du gel obtenu.

Le procédé, selon la présente invention, permet d'obtenir de façon constante une qualité très élevée du gel tout en faisant appel à des techniciens moins expérimentés, ce qui réduit le coût de fabrication.

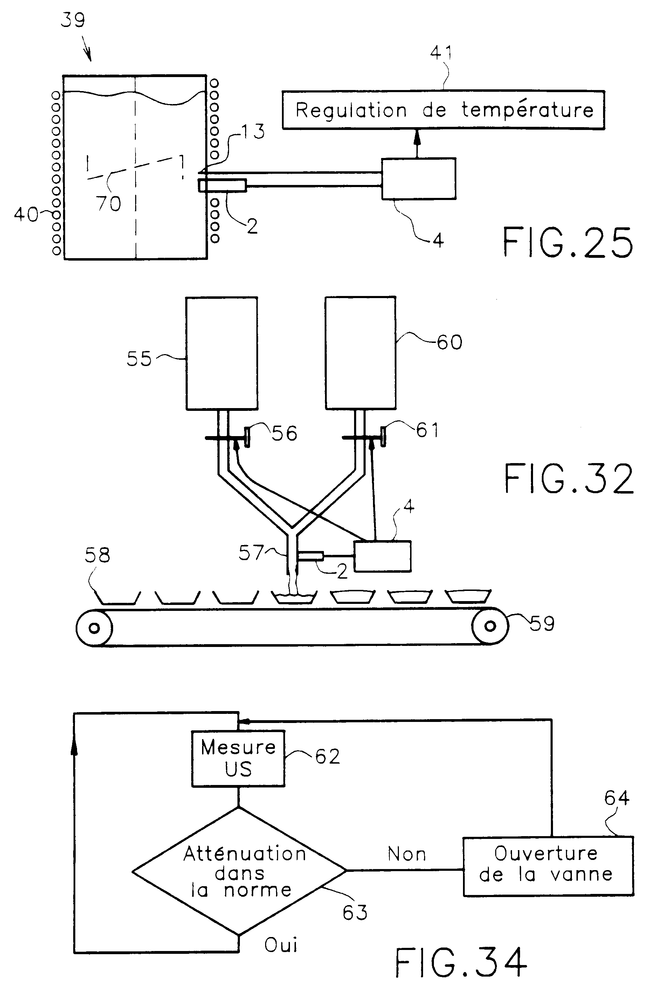

Sur la figure 32, l'on peut voir un dispositif de moulage, selon la présente invention, comportant une première cuve 55 reliée par une première vanne 56 à un dispositif de remplissage 57 de barquettes 58 circulant sur un convoyeur 59 et une seconde cuve 60 reliée par une seconde vanne 61 au dispositif de remplissage 57.

Une sonde à ultrasons 2 est reliée à une unité 4 de génération et de traitement de signal qui commande l'ouverture plus ou moins importante des vannes 56 et 61.

L'industrie agro-alimentaire a toujours cherché à obtenir une composition uniforme des produits de remplissage des barquettes se traduisant par une viscosité donnée. Si le produit contenu dans la cuve 55 est très épais, l'on rajoute de l'eau contenue dans la cuve 60.

L'organigramme du procédé selon la présente invention est illustré sur la figure 34.

En 62, l'on mesure l'atténuation relative des ultrasons au niveau des dispositifs de remplissage 57.

On va en 63.

En 63, l'on vérifie si l'atténuation correspond à la norme désirée.

Si oui, on va en 62.

Si non, on va en 64.

En 64, l'on commande l'ouverture de la vanne 61 permettant de rajouter de l'eau. Après avoir rajouté la quantité désirée d'eau, l'on ferme la vanne 61.

On va en 62.

Sur la figure 33, l'on peut voir une courbe 64 représentant l'amplitude du signal capté exprimé en milli volts en fonction du temps exprimé en secondes et une courbe 65 représentant le retard des signaux captés en fonction du temps exprimé en secondes. La courbe 64 présente une variation importante en 66 et 67 correspondant à la mise en service d'une nouvelle cuve 55 de la figure 32, la cuve précédente étant vide.

Ainsi, une variation de l'atténuation des ultrasons dans le milieu permet d'en déterminer la viscosité, tandis que la variation de la vitesse de propagation des ultrasons dans le milieu mesuré exprimée par la courbe 65 ne donne pas de résultats significatifs.

Il est bien entendu que l'invention n'est pas limitée au contrôle des constituants d'un mélange, mais s'applique à tout réglage de paramètres physiques et/ou chimiques sur une chaîne de production et notamment au réglage de la température, de la pression, de l'acidité, de la vitesse d'écoulement, du brassage ou de l'agitation, de l'irradiation avec des ondes électromagnétiques, de la diffusion ou du dépôt d'un produit.

De même, l'invention n'est pas limitée à une insonification non destructive du produit en cours de fabrication. Si cela est désiré, les ondes ultrasonores de fortes amplitudes peuvent apporter au milieu une énergie suffisante pour apporter des modifications au produit.

Cette vitesse de propagation est principalement reliée à la température par une formule du type $V = A + B, T + C, T^2 + ...$ , A, B et C étant des constantes et T étant la température.

La présente invention permet de détecter et de quantifier la présence de morceaux ou d'air (foisonnement) dans un flux de matières, notamment à travers un tuyau. Il est, par exemple, possible de réguler un processus de fabrication industrielle d'un produit pour obtenir la quantité et la taille des morceaux désirées ou la quantité d'air désirée à une étape de fabrication de ce produit.

La présente invention permet de suivre l'évolution d'un produit en cours de fabrication pour s'assurer que les normes de fabrication sont respectées, pour minimiser le coût de revient en diminuant au strict nécessaire le temps de fabrication, l'utilisation de matières premières et la consommation énergétique, en maximisant la qualité du produit fabriqué et en détectant le plus tôt possible (et en remédiant) à toute apparition d'un défaut risquant de compromettre les propriétés organoleptiques du produit.

L'invention s'applique à toute industrie nécessitant la caractérisation ou la mesure de la texture, et notamment à l'industrie alimentaire, pharmaceutique, cosmétologique, plastique et aux industries de peinture.

L'invention s'applique particulièrement à la fabrication de fromages.

**Revendications**

1.  Procédé de détection d'un changement de phases et/ou de caractérisation de la phase d'un produit (16) comportant une étape d'insonification du produit avec des ondes ultrasonores et une étape de réception du rayonnement acoustique du produit insonifié, caractérisé en ce qu'il comporte les étapes consistant à :

    - disposer un transducteur d'émission/réception ou un transducteur d'émission et le transducteur de réception à l'extérieur d'un contenant du produit à étudier ;
    - assurer un couplage acoustique entre le ou les transducteurs et le produit à étudier ;
    - analyser le signal reçu du produit à étudier et le comparer à un modèle ;
    - en déduire la phase ou le mélange de phases du produit.

2.  Procédé de détection d'un changement de phases et/ou de caractérisation de la phase d'un produit (16) selon la revendication 1 comportant les étapes consistant à :

- analyser le signal reçu du produit à étudier et le comparer à un modèle ;
- en déduire la phase ou le mélange de phases du produit, caractérisé en ce qu'il comporte une étape de sélection automatique d'une fenêtre temporelle dans le signal capté ayant le meilleur rapport signal/bruit.

3. Procédé selon la revendication 1 ou 2, caractérisé en ce que l'on insonifie le produit avec une onde ultrasonore en mode de vibration longitudinale.

4. Procédé selon la revendication 1, 2 ou 3, caractérisé en ce qu'il comporte une étape de mesure de l'atténuation des ondes ultrasonores dans le produit en fonction du temps de réception, c'est-à-dire en fonction de la profondeur de pénétration des ondes ultrasonores dans le produit.

5. Procédé selon la revendication 1, 2 ou 3, caractérisé en ce qu'il comporte une étape d'émission d'ondes ultrasonores en direction d'un miroir acoustique (31) immergé dans le produit (16) et une étape de réception des ondes ultrasonores réfléchies sur le miroir (31).

6. Procédé selon la revendication 2, 3, 4 ou 5, caractérisé en ce qu'il comporte des étapes consistant à :

- calculer le logarithme népérien (28) de l'enveloppe du signal reçu ;
- calculer la régression linéaire R du logarithme (28) de l'enveloppe (17) du signal reçu pour les diverses parties de cette enveloppe ;
- déterminer la fenêtre temporelle (29) de l'enveloppe (17) correspondant au maximum du coefficient de régression linéaire R du logarithme (28) de l'enveloppe (17) ;
- déterminer l'absorption des ondes ultrasonores uniquement dans ladite fenêtre temporelle (29).

7. Procédé selon l'une quelconque des revendications précédentes, caractérisé en ce qu'il comporte une étape de comparaison de l'énergie reçue en fonction du temps à un modèle ou à une échelle d'étalonnage.

8. Procédé selon l'une quelconque des revendications précédentes, caractérisé en ce qu'il comporte une étape de comparaison du spectre de puissance des ondes captées à un modèle ou à une échelle d'étalonnage.

9. Procédé selon l'une quelconque des revendications précédentes, caractérisé en ce qu'il comporte des étapes consistant à :

a) numériser le signal reçu sur N points,

b) choisir une succession de n points du signal, n étant inférieur à N,

c) calculer de la transformée de Fourrier, de préférence par l'algorithme TFR, de la succession de n points,

d) mesurer l'amplitude du résultat de calcul de la transformée de Fourrier,

e) répéter les étapes b) à d) pour une pluralité de successions de points.

10. Procédé selon l'une quelconque des revendications précédentes, caractérisé en ce qu'il comporte une étape de mesure de la température et de correction des résultats en fonction de la valeur mesurée de la température.

11. Procédé selon l'une quelconque des revendications précédentes, caractérisé en ce qu'il comporte une étape de détection automatique de l'apparition d'un défaut dans un produit, notamment d'un défaut risquant de compromettre les propriétés organoleptiques d'un produit alimentaire.

12. Procédé de fabrication d'un produit, notamment d'un produit alimentaire, caractérisé en ce qu'il met en oeuvre un procédé de détection de changement de phases et/ou de caractérisation de phases d'un produit selon l'une quelconque des revendications précédentes.

13. Procédé selon la revendication 12, caractérisé en ce qu'il comporte une étape de détection automatique de l'apparition d'un défaut dans un produit, notamment d'un défaut risquant de compromettre les propriétés organoleptiques d'un produit alimentaire et une étape de rétroaction sur au moins un paramètre de fabrication pour faire disparaître ou, tout au moins diminuer, les conséquences du défaut détecté.

14. Procédé selon la revendication 12 ou 13, caractérisé en ce qu'il comporte les étapes consistant à :

- mélanger deux produits ;
- déterminer la proportion des produits, morceaux ou air (foisonnement), ou les propriété physiques du mélange obtenu, notamment la viscosité, par le procédé selon l'une quelconque des revendications 1 à 11 ;
- modifier les proportions des produits pour obtenir le mélange désiré.

15. Procédé selon la revendication 12 ou 13, caractérisé en ce qu'il comporte une étape de prise de gel d'un produit dont la durée ainsi que la température à laquelle est porté le produit sont asservies par un procédé selon l'une quelconque des revendications 1 à 8.

**16.** Dispositif pour la mise en oeuvre d'un procédé selon l'une quelconque des revendications 1 à 15, caractérisé en ce qu'il comporte une sonde d'émission (2, 2') et de réception (2, 2") ultrasonore, disposée(s) à l'extérieur d'un contenant (32) du produit à étudier (16) et reliée(s) à un émetteur (5) pour générer un signal électrique converti en une onde ultrasonore par la sonde (2) et un récepteur (6) des ondes ultrasonores captées par la sonde (2, 2") ainsi que des moyens de traitement du signal (7, 8) pour détecter un changement de phases et/ou pour caractériser la phase d'un produit.

**17.** Dispositif selon la revendication 16, caractérisé en ce qu'il comporte une sonde unique (2) d'émission et de réception susceptible de recevoir l'énergie rétro-diffusée par le produit (16) insonifié.

**18.** Dispositif selon la revendication 16, caractérisé en ce qu'il comporte une sonde unique d'émission de réception ainsi qu'un miroir acoustique (31) pour réfléchir vers la sonde (2) des ondes ultrasonores.

**19.** Dispositif selon la revendication 16, caractérisé en ce qu'il comporte une sonde (2') d'émission des ondes ultrasonores et une sonde (2") de réception des ondes ultrasonores placées en vis à vis de part et d'autre du produit à insonifier.

**20.** Dispositif pour la mise en oeuvre d'un procédé selon l'une quelconque des revendications 16 à 19, caractérisé en ce que lesdits moyens de traitement du signal (7, 8) comportent des moyens de sélection automatique d'une fenêtre temporelle (29) ayant le meilleur rapport signal/bruit.

**Claims**

**1.** Process for detecting a phase change and/or for characterising the phase of a product (16), comprising a step of sonicating the product with ultrasound waves and a step of receiving the acoustic radiation from the sonicated product, characterised in that it comprises the steps of:

- placing an emitting/receiving transducer or an emitting transducer and the receiving transducer outside a container holding the product which is to be studied;
- providing an acoustic coupling between the or each transducer and the product which is to be studied;
- analysing the signal received from the product which is to be studied and comparing it with a model;
- deducing from this the phase or mixture of phases of the product.

**2.** Process for detecting a phase change and/or for characterising the phase of a product (16) according to claim 1, comprising the steps of:

- analysing the signal received from the product which is to be studied and comparing it with a model;
- deducing from this the phase or mixture of phases of the product,
    characterised in that it comprises a step of automatically selecting a time window in the signal picked up which has the best signal-to-noise ratio.

**3.** Process according to claim 1 or 2, characterised in that the product is sonicated with an ultrasound wave in longitudinal vibration mode.

**4.** Process according to claim 1, 2 or 3, characterised in that it comprises a step of measuring the attenuation of the ultrasound waves in the product as a function of the reception time, i.e. as a function of the depth of penetration of the ultrasound waves in the product.

**5.** Process according to claim 1, 2 or 3, characterised in that it comprises a step of emitting ultrasound waves towards an acoustic mirror (31) immersed in the product (16) and a step of receiving the ultrasound waves reflected by the mirror (31).

**6.** Process according to claim 2, 3, 4 or 5, characterised in that it comprises steps of:

- calculating the Napierian logarithm (28) of the envelope of the signal received;
- calculating the linear regression R of the logarithm (28) of the envelope (17) of the signal received for the various parts of this envelope;
- determining the time window (29) of the envelope (17) corresponding to the maximum coefficient of linear regression R of the logarithm (28) of the envelope (17);
- determining the absorption of the ultrasound waves solely in said time window (29).

**7.** Process according to any one of the preceding claims, characterised in that it comprises a step of comparing the energy received as a function of time with a model or a calibrating scale.

**8.** Process according to any one of the preceding claims, characterised in that it comprises a step of comparing the power spectrum of the waves picked up with a model or a calibrating scale.

**9.** Process according to any one of the preceding claims, characterised in that it comprises steps consisting of:

a) digitising the signal received over N points,

b) choosing a succession of n points of the signal, n being less than N,

c) calculating the Fourrier transform, preferably by the TFR algorithm, of the succession of n points,

d) measuring the amplitude of the result of the calculation of the Fourrier transform,

e) repeating steps b) to d) for a plurality of successions of points.

**10.** Process according to any one of the preceding claims, characterised in that it comprises a step of measuring the temperature and correcting the results as a function of the measured value of the temperature.

**11.** Process according to any one of the preceding claims, characterised in that it comprises a step of automatically detecting the appearance of a defect in a product, notably a defect which could compromise the organoleptic properties of a food product.

**12.** Process for manufacturing a product, notably a food product, characterised in that it uses a process for detecting phase changes and/or for characterising the phases of a product, according to any one of the preceding claims.

**13.** Process according to claim 12, characterised in that it comprises a step of automatically detecting the appearance of a defect in a product, notably a defect which could compromise the organoleptic properties of a food product, and a feedback step acting on at least one manufacturing parameter in order to eliminate or at least reduce the consequences of the defect detected.

**14.** Process according to claim 12 or 13, characterised in that it comprises the steps of:

- mixing two products;
- determining the proportion of the products, pieces or air (aeration), or the physical properties of the mixture obtained, notably the viscosity, by the process according to any one of claims 1 to 11;
- modifying the proportions of the products to obtain the desired mixture.

**15.** Process according to claim 12 or 13, characterised in that it comprises a step of gelatinising a product, the duration of which and the temperature to which the product is brought being automatically controlled by a process according to any one of claims 1 to 8.

**16.** Apparatus for carrying out a process according to any one of claims 1 to 15, characterised in that it comprises an ultrasonic emitter probe (2, 2') and receiving probe (2, 2''), disposed outside a container (32) holding the product (16) which is to be examined and connected to an emitter (5) for generating an electrical signal converted into an ultrasound wave by the probe (2) and a receiver (6) for the ultrasound waves picked up by the probe (2, 2'') and means for processing the signal (7, 8) in order to detect a change of phase and/or characterise the phase of a product.

**17.** Apparatus according to claim 16, characterised in that it comprises a single emitting and receiving probe (2) capable of receiving the energy fed back by the sonicated product (16).

**18.** Apparatus according to claim 16, characterised in that it comprises a single emitting and receiving probe and an acoustic mirror (31) for reflecting ultrasound waves back to the probe (2).

**19.** Apparatus according to claim 16, characterised in that it comprises a probe (2') for emitting ultrasound waves and a probe (2'') for receiving ultrasound waves which are placed facing each other on either side of the product which is to be sonicated.

**20.** Apparatus for carrying out a process according to any one of claims 16 to 19, characterised in that the signal processing means (7, 8) comprise means for automatically selecting a time window (29) having the best signal-to-noise ratio.

**Patentansprüche**

**1.** Verfahren zur Ermittlung einer Änderung der Phasen und/oder der Phasencharakteristik eines Produkts (16), umfassend eine Beschallung des Produkts mit Ultraschallwellen und Empfangen der akustischen Strahlung von dem beschallten Produkt, gekennzeichnet durch folgende Verfahrensschritte:

- Anordnen eines Sende-/Empfangstransduktors oder eines Sendetransduktors und eines Empfangstransduktors außerhalb eines Behälter für das zu untersuchende Produkt;

- Sicherstellen einer akustischen Kopplung zwischen dem oder den Transduktoren und dem zu untersuchenden Produkt;

- Analysieren des von dem zu untersuchenden Produkt empfangenen Signals und vergleichen des Signals mit einem Muster bzw. einer Referenz; und

- daraus Berechnen bzw. Ableiten der Phase oder einer Phasenmischung des Produkts.

2. Verfahren zur Ermittlung einer Änderung der Phasen und/oder der Phasencharaktzeristik eines Produktes (16) gemäß Anspruch 1, umfassend folgende Verfahrensschritte:

   - Analysieren des von dem zu untersuchenden Produkt empfangenen Signals und Vergleichen des Signals mit einem Muster bzw. einer Referenz;
   - daraus Ableiten bzw. Errechnen der Phase oder Phasenmischung des Produkts, gekennzeichnet durch eine automatische Selektion eines zeitlichen Fensters in dem erhaltenen Signal, das die wesentliche Aussage des Signals/Tons enthält.

3. Verfahren nach Anspruch 1 oder 2, dadurch gekennzeichnet, dass das Produkt mit Ultraschallwellen mit einem longitudinalen Schwingungsmodus bestrahlt wird.

4. Verfahren nach Anspruch 1, 2 oder 3, dadurch gekennzeichnet, daß die Dämpfung der Ultraschallwellen in dem Produkt als Funktion der Empfangszeit, d.h. als Funktion der Eindringtiefe der Ultraschallwellen in das Produkt gemessen wird.

5. Verfahren nach Anspruch 1, 2 oder 3, dadurch gekennzeichnet, daß die Ultraschallwellen in Richtung eines in das Produkt (16) eingetauchten akustischen Spiegels (31) emittiert und die von diesem Spiegel (31) reflektierten Ultraschallwellen empfangen werden.

6. Verfahren nach Anspruch 2, 3, 4 oder 5, gekennzeichnet durch folgende Verfahrensschritte:

   - Errechnen des Néperschen Logarithmus (28) aus der Hüllkurve des empfangenen Signals;

   - Errechnen der linearen Regression R des Logarithmus (28) der Hüllkurve (17) des empfangenen Signals für unterschiedliche Abschnitte dieser Hüllkurve;

   - Ermitteln eines zeitlichen Fensters (29) der Hüllkurve (17), das mit dem Maximum des Koeffizienten der linearen Regression R des Logarithmus (28) der Hüllkurve (17) korrespondiert;

- Ermitteln der Absorption der Unterschallwellen allein in dem zeitlichen Fenster (29).

7. Verfahren nach einem der vorgehenden Ansprüche, dadurch gekennzeichnet, dass die empfangenen Energie als Funktion der Zeit mit einem Muster bzw. einer Referenz oder mit einer Eichskala verglichen wird.

8. Verfahren nach einem der vorgehenden Ansprüche, dadurch gekennzeichnet, dass das Energiespektrum der empfangenen Wellen mit einem Muster bzw. einer Referenz oder einer Eichskala verglichen wird.

9. Verfahren nach einem der vorhergehenden Ansprüche, gekennzeichnet durch folgende Schritte:

   a) Nummerieren des Empfangssignals über N Punkte
   b) Wählen einer Abfolge von n Punkten des Signals, wobei n kleiner als N ist,
   c) Errechnen der Fourier-Transformation, vorzugsweise über den Algorithmus TFR der Abfolge der n Punkte
   d) Messen der Amplitude des Resultats aus der Berechnung der Fourier-Transformation,
   e) Wiederholen der Schritte b) bis d) für eine Mehrzahl von Punkte-Abfolgen.

10. Verfahren nach einem der vorhergehenden Ansprüche, dadurch gekennzeichnet, dass die Temperatur gemessen und die Resultate als Funktion des gemessenen Temperaturwertes korrigiert werden.

11. Verfahren nach einem der vorhergehenden Ansprüche, gekennzeichnet durch einen Schritt zur automatischen Ermittlung des Auftretens eines Fehlers in dem Produkt, insbesondere eines riskanten Fehlers, der die organoleptischen Eigenschaften eines Lebensmittelproduktes beeinträchtigen könnte.

12. Verfahren zur Herstellung eines Produkts, insbesondere eines Lebensmittels, gekennzeichnet durch Vorsehen eines Verfahrens zur Ermittlung der Änderung der Phasen und/oder der Phasencharakteristik eines Produktes nach einem der vorhergehenden Ansprüche.

13. Verfahren nach Anspruch 12, gekennzeichnet durch einen Schritt zum automatischen Detektieren des Auftretens eines Fehlers in dem Produkt, insbesondere eines riskanten Fehlers, der die organoleptischen Eigenschaften eines Lebensmittels beeinträchtigen könnte und eines Schrittes zur Rückkoppelung zumindest eines Herstellungsparameters, um die Konsequenzen des detektierten Fehlers zu eliminieren oder zumindest zu verringern.

14. Verfahren nach Anspruch 12 oder 13, umfassend folgende Schritte:

   - Mischen zweier Produkte;

   - Ermitteln des Anteils der Produkte, der Bruchstücke oder Luft (Aufquellen) oder der physischen Eigenschaften der erhaltenen Mischung, insbesondere die Viskosität gemäß dem Verfahren nach einem der vorhergehenden Ansprüche 1 bis 11;

   - Verändern der Anteile des Produkts, um die gewünschte Mischung zu erhalten.

15. Verfahren nach Anspruch 12 oder 13, dadurch gekennzeichnet, dass die Liegezeit des Gels eines Produkts als auch die Temperatur, auf das Produkt gebracht wird, durch ein Verfahren nach einem der Ansprüche 1 bis 8 gesteuert bzw. geregelt werden.

16. Vorrichtung zur Durchführung eines Verfahrens nach einem der Ansprüche 1 bis 15, dadurch gekennzeichnet, dass sie eine Sonde zur Emission (2,2') und zum Empfang (2,2'') von Ultraschallwellen enthält, die außerhalb eines Behälters (32) für ein zu untersuchendes Produkt (16) angeordnet und mit einem Sender zum Generieren eines elektrischen Signals verbunden sind, das durch die Sonde (2) in eine Ultraschallwelle umgewandelt wird, und mit einem Empfänger (6) für die durch die Sonde (2,2'') empfangenen Ultraschallwellen als auch eine Vorrichtung zur Verarbeitung des Signals (7, 8) zum Detektieren einer Änderung der Phasen und/oder zum Charakterisieren der Phase eines Produkts.

17. Vorrichtung nach Anspruch 16, dadurch gekennzeichnet, dass sie eine einzige Sonde (2) zum Senden als auch zum Empfangen aufweist, die in der Lage ist, die von dem bestrahlten Produkt (16) zurückgestrahlt und/oder gestreute Energie zu empfangen.

18. Vorrichtung nach Anspruch 16, dadurch gekennzeichnet, dass sie eine einzige Sonde zum Senden und Empfangen aufweist als auch einen akustischen Spiegel (31) zum Reflektieren der Ultraschallwellen in Richtung auf die Sonde (2).

19. Vorrichtung nach Anspruch 16, dadurch gekennzeichnet, dass sie eine Sonde (2') zum Senden der Ultraschallwellen als auch eine Sonde (2'') zum Empfang der Ultraschallwellen aufweist, die einerseits einander gegenüber und andererseits zum zu bestrahlenden Produkt hin angeordnet ist.

20. Vorrichtung zur Durchführung des Verfahrens nach einem der Ansprüche 16 bis 19, dadurch gekennzeichnet, dass die Vorrichtung zur Verarbeitung des Signals (7, 8) eine Vorrichtung zur automatischen Selektion eines zeitlichen Fensters (29) aufweist, das den wesentlichen Informationsanteil des Signals/Tons aufweist.

FIG.1

FIG.2

FIG.3

FIG.4

FIG.5

FIG.6

FIG.7

FIG.8

FIG.9

FIG.10

FIG.11

FIG.12

FIG.13

EP 0 577 511 B1

26

21 22

Sonde → Récepteur → A/N → Cadrage (t) → TFR — 23

2 6 19

a+jb — 24

7

TFR⁻¹ — 25

20

**FIG.14**

Enveloppe

26 27

2 26 19

Sonde → Récepteur → → Enveloppe

6

7 20

**FIG.15**

FIG.16

FIG.17

FIG.18

FIG.19

FIG.20

FIG.23

FIG.24

FIG.21

FIG.22

EP 0 577 511 B1

FIG.28

FIG.30

FIG.27

FIG.29

FIG.31

Chargement des cuves — 46
Brassage+Montée en température — 47    Etape 1
Mesure US Mesure de la température — 48
La texturation est terminée — 49
Non
Oui
Pallier de temperature — 50    Etape 2
Mesure de la texture du gel — 51
Maintien de la température — 75
La texture desirée est atteinte — 52
Non
Oui    Etape3
Descente en température — 53
Moulage — 54

FIG.33

FIG.26

39

40

13

70

2

41

Regulation de température

4

## FIG.25

55

60

56

61

## FIG.32

4

57

2

58

59

Mesure US

62

64

Atténuation dans la norme

63

Non

Ouverture de la vanne

Oui

## FIG.34

FIG.35

FIG.36